# EUROPEAN PATENT APPLICATION

(11) **EP 3 480 297 A1**
(43) Date of publication of application: **08.05.2019**
(21) Application number: 17819815.6
(22) Date of filing: 08.06.2017
(51) Int. Cl.: C12N 5/071, A01K 67/027, A61K 35/22, A61L 27/36, A61L 27/38, A61P 13/12, C12N 15/09

(54) **ORGAN PRODUCTION METHOD**

(30) Priority: 29.06.2016 JP 2016129392
(71) Applicant: Yokoo, Takashi, Tokyo 105-8461 (JP); Tes Holdings Co., Ltd., Tokyo 110-0015 (JP)
(72) Inventor: YOKOO, Takashi, Tokyo 105-8461 (JP); YAMANAKA, Shuichiro, Tokyo 105-8461 (JP)
(74) Representative: Schnappauf, Georg
(86) International application number: PCT/JP2017/021301
(87) International publication number: WO 2018/003450

(57) **Abstract**

The present invention provides an organ production method including a step of tissue-specifically removing a first portion of an organ of a non-human animal partway through development thereof; a step of transplanting, into a region from which the first portion has been removed, an organ precursor cell which is allogeneic or xenogeneic to the non-human animal; and a step of advancing development of the organ, which is a step in which the transplanted organ precursor cell is differentiated and matured to form a part of the organ.

## Description

### Technical Field

The present invention relates to an organ production method. More specifically, the present invention relates to an organ production method, an organ, a non-human animal, a kit for organ production, and a medical drug for organ regeneration. Priority is claimed on Japanese Patent Application No. 2016-129392, filed on June 29, 2016, the content of which is incorporated herein by reference.

### Background Art

At present, various tissue-specific precursor cells or tissue stem cells can be induced from pluripotent stem cells such as iPS cells and ES cells (refer to, for example, Non-Patent Document 1). However, for example, many organs including kidney, for example, have low self-repairing ability, and therefore expression of functions thereof depends on a complicated structure composed of various kinds of cells. For this reason, current techniques have not yet reached a stage where an organ having complicated three-dimensional structures can be regenerated from organ precursor cells.

### Citation List

### Non-Patent Literature

[Non-Patent Document 1] Takasato M, et al., Directing human embryonic stem cell differentiation towards a renal lineage generates a self-organizing kidney, Nat. Cell Biol., 16 (1), 118-126, 2014.

### Summary of Invention

### Technical Problem

An object of the present invention is to provide a technique for producing an organ from organ precursor cells.

### Solution to Problem

The present invention includes the following aspects.
(1) An organ production method including a step of tissue-specifically removing a first portion of an organ of a non-human animal partway through development thereof; a step of transplanting an organ precursor cell which is allogeneic or allogeneic to the non-human animal into the organ; and a step of advancing development of the organ, in which the transplanted organ precursor cell is differentiated and matured to form a part of the organ.
(2) The organ production method according to (1), further including a step of tissue-specifically removing a portion different from the first portion of the organ partway through the development thereof; and a step of transplanting an organ precursor cell which is allogeneic or xenogeneic to the non-human animal into the organ.
(3) The organ production method according to (1) or (2), in which the organ precursor cell is a human cell.
(4) The organ production method according to (1) or (2), in which the kidney precursor cell is a non-human animal cell.
(5) The organ production method according to (4), in which the non-human animal cell is a cat cell.
(6) The organ production method according to any one of (1) to (5), in which the non-human animal is a pig.
(7) The organ production method according to any one of (1) to (5), in which the non-human animal is a mouse.
(8) The organ production method according to any one of (1) to (5), in which the non-human animal is a cat.
(9) An organ produced by the production method according to any one of (1) to (8).
(10) An organ including: a cell derived from a non-human animal; and a cell which is allogeneic or xenogeneic to the non-human animal, in which a proportion of the cell which is allogeneic or xenogeneic to the non-human animal is 70% by mass or more.
(11) The organ according to (10), in which the cell which is allogeneic or xenogeneic to the non-human animal is a human cell.
(12) The organ according to (10), in which the cell which is allogeneic or xenogeneic to the non-human animal is a non-human animal cell.
(13) The organ according to (12), in which the non-human animal cell is a cat cell.
(14) A non-human animal including the organ according to any one of (9) to (13).
(15) A genetically modified non-human animal for producing the organ according to any one of (9) to (13), from which a first portion of the organ partway through development thereof can be tissue-specifically removed.
(16) The genetically modified non-human animal according to (15), from which a portion different from the first portion of the organ can be further tissue-specifically removed.
(17) The genetically modified non-human animal according to (15) or (16), which is a genetically modified pig.
(18) A kit for organ production including an organ of a non-human animal partway through development thereof; a medicine which tissue-specifically removes a first portion of the organ; and an organ precursor cell which is allogeneic or xenogeneic to the non-human animal.
(19) The kit for organ production according to (18), further including a medicine which tissue-specifically removes a portion different from the first portion of the organ partway through the development thereof.
(20) A medical drug for organ regeneration, including an organ of a non-human animal partway through development thereof; a medicine which tissue-specifically removes a first portion of the organ; and an organ precursor cell.
(21) The medical drug for organ regeneration according to (20), further including a medicine which tissue-specifically removes a portion different from the first portion of the organ.

### Advantageous Effects of Invention

According to the present invention, it is possible to provide a technique for producing an organ from organ precursor cells.

### Brief Description of Drawings

FIG. 1 is a schematic diagram showing a fetus of a mouse at around day 11 of embryonic development.
(a) to (c) of FIG. 2 are schematic diagrams for explaining how a kidney is developed.
FIG. 3 is a photograph showing a result of immunostaining of a kidney tissue sample in Experimental Example 3.
FIG. 4 is a photograph showing a result of immunostaining of a kidney tissue sample in Experimental Example 4.
(a) and (b) of FIG. 5 are photographs of a kidney regenerated in a mouse body in Experimental Example 5.
(a) and (b) of FIG. 6 are microphotographs of tissue section samples of the kidney regenerated in the mouse body in Experimental Example 5.
(a) to (c) of FIG. 7 are photographs showing results of immunostaining of a tissue sample of a metanephros in Experimental Example 6.

### Description of Embodiments

### [Organ Production Method]

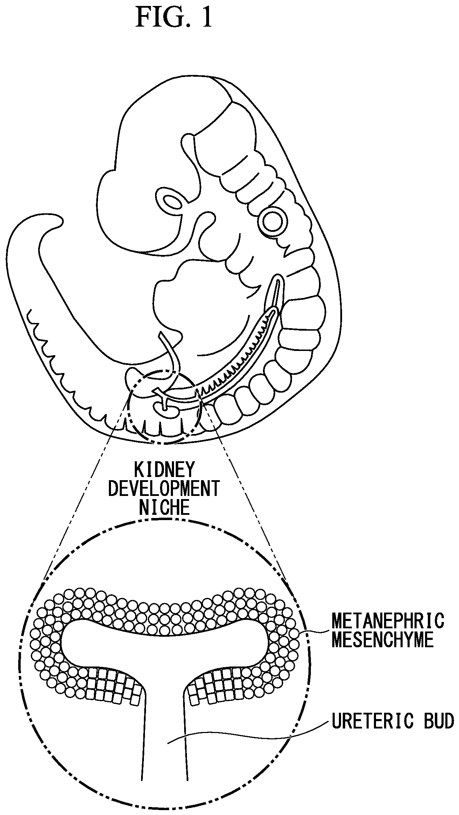

The development of an organ by will be explained using a kidney as an example. FIG. 1 is a schematic diagram showing a fetus of a mouse at around day 11 of embryonic development. A kidney is formed through three stages of pronephros, mesonephros, and metanephros. Among these, the pronephros and mesonephros are degenerated later. A kidney functioning in an adult body of mammals is the metanephros.

As shown in FIG. 1, the kidney is formed by an interaction between an ureteric bud and a metanephric mesenchyme surrounding the ureteric bud. The ureteric bud constitutes from collecting ducts to a ureter, and nephron precursor cells contained in the metanephric mesenchyme are the origin of glomeruli and kidney tubules. As described above, an interaction between a plurality of tissues is important for the development of an organ having a complicated three-dimensional structure. In the present specification, an organ development region including such plurality of tissues is called an organ development niche.

In one embodiment, the present invention provides an organ production method including a step (i) of tissue-specifically removing a first portion of an organ of a non-human animal partway through the development thereof; a step (ii) of transplanting, into the organ, an organ precursor cell which is allogeneic or xenogeneic to the non-human animal; and a step (iii) of advancing development of the organ, which is a step in which the transplanted organ precursor cell is differentiated and matured to form a part of the organ.

In the related art, efficiency of a cell transplant into the organ development niche was extremely poor in terms of fixation. On the other hand, as described later in Examples, according to a production method of the present embodiment, transplanted organ precursor cells can be engrafted with high efficiency. In addition, the transplanted organ precursor cells take over a development program of a host and autonomously advance complicated differentiation induction to form a part of the organ.

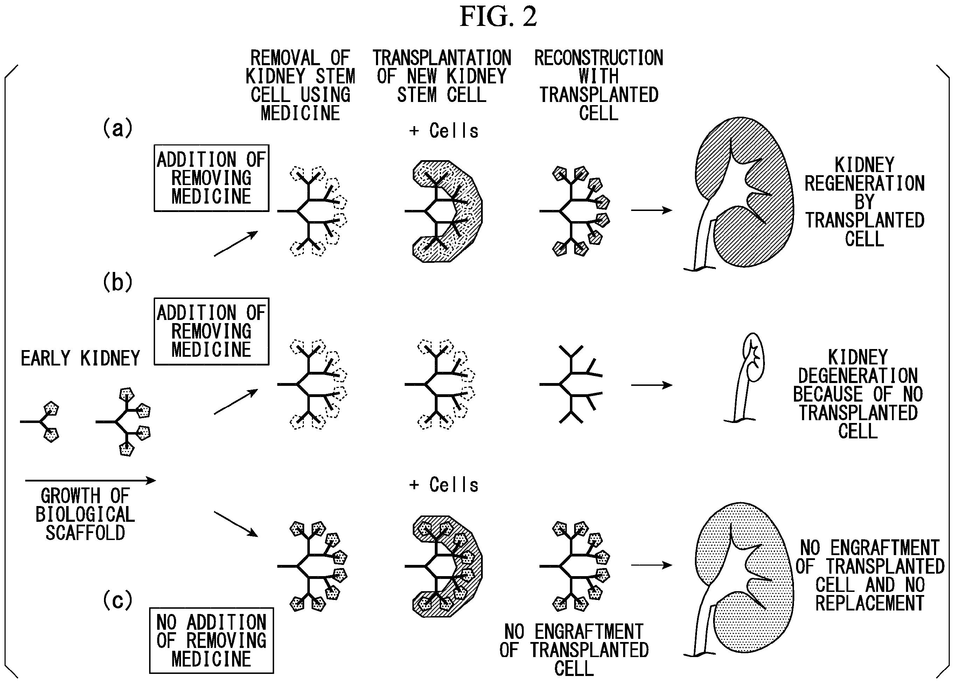

An organ production method of the present embodiment will be described in more detail with reference to FIG. 2 while using the kidney as an example. (a) to (c) of FIG. 2 are schematic diagrams for explaining how a kidney is developed. Among these, (a) of FIG. 2 corresponds to the organ production method of the present embodiment.

In (a) of FIG. 2, a metanephric mesenchyme of an early kidney of a non-human animal embryo is removed by adding a removing medicine. The addition results in a state in which kidney stem cells derived from the host do not exist, that is, a state in which the niche is empty. Subsequently, new kidney precursor cells (kidney stem cells) are transplanted into this kidney development niche. Then, the transplanted kidney precursor cells are fixed and take over the development program of the host to proceed the development of the kidney. As a result, the kidney is regenerated by the transplanted kidney precursor cells.

Meanwhile, in (b) of FIG. 2, a metanephric mesenchyme of an early kidney of a non-human animal embryo is removed by adding a removing medicine. The addition results in a state in which kidney stem cells derived from the host do not exist, that is, a state in which the niche is empty. In (b) of FIG. 2, a transplantation of new kidney precursor cells is not carried out. Even if the development of the kidney progresses in this state, the kidney will be degenerated.

In addition, in (c) of FIG. 2, new kidney precursor cells are transplanted into the metanephric mesenchyme of the early kidney of the non-human animal embryo while remaining the kidney stem cells derived from the host. However, competition by the existing cells derived from the host which occupies the kidney development niche is strong, and thus the transplanted kidney precursor cells are not fixed. As a result, a kidney composed of the cells derived from the host is formed.

Hereinafter, each step of the organ production method of the present embodiment will be described in detail.

### (Step (i))

In the present step, a first portion of an organ of a non-human animal partway through the development thereof is tissue-specifically removed. The non-human animal is not particularly limited, and may be, for example, a pig. Pigs are suitable for a transplantation into a human because of a size of organs thereof, and genetic modification techniques have also been established. Alternatively, the non-human animal may be a mouse. Mice are easy to use because genetic modification techniques and various experimental systems have been established. Alternatively, the non-human animal may be a cat. Cats are one of the most common animals as a pet, but are an animal having a very high incidence of chronic kidney disease. For example, there are reports that about 30% of the cause of cat death is chronic kidney failure and 50% or more of cats die or are euthanized due to a deterioration in kidney functions. For this reason, there is a potential demand for the kidney transplantation as one of life-prolonging techniques for cats suffering from kidney disease.

In the production method of the present embodiment, the organ is not particularly limited, and examples thereof include kidney, heart, liver, pancreas, adrenal gland, intestinal tract, and the like. As will be described later in Examples, according to the production method of the present embodiment, it is possible to produce an organ having a complicated three-dimensional structure, which could not be artificially produced in the related art.

In the production method of the present embodiment, the first portion of the organ means a portion of a plurality of tissues in which a development program progresses while interacting in the development of the organ. The tissue-specific removal of the first portion of the organ is not particularly limited as long as the first portion of the organ can be tissue-specifically removed, and can be performed by using, for example, a genetic recombination technique.

A more specific method of tissue-specifically removing a metanephric mesenchyme in a metanephros will be described by using a mouse system as an example. Examples of the method of tissue-specific removal of the metanephric mesenchyme include a method in which an iDTR mouse expressing a diphtheria toxin receptor (DTR) in a Cre recombinase activity-dependent manner is crossed with Six2-Cre mouse in which a Cre recombinase gene is introduced into downstream of a promoter of Six2; and the diphtheria toxin is brought into contact with the metanephros tissue of a progeny to be obtained.

The iDTR mouse has a transcription termination sequence flanked by two loxP sequences upstream of a gene encoding the diphtheria toxin receptor. For this reason, the diphtheria toxin receptor is not expressed in this state. However, if the transcription termination sequence flanked by the two loxP sequences is removed by the Cre recombinase, the diphtheria toxin receptor is expressed.

Since mice do not have the diphtheria toxin receptor, cells of mice are not killed even if the diphtheria toxin is brought into contact with the cells thereof. However, it is known that when the diphtheria toxin is brought into contact with cells of a mouse expressing the diphtheria toxin receptor, the cells are killed. In the above-described example, this phenomenon is utilized.

First, if the iDTR mouse is crossed with the Six2-Cre mouse specifically expressing the Cre recombinase in the metanephric mesenchyme, a mouse tissue-specifically expressing the diphtheria toxin receptor in the metanephros tissue among progenies to be obtained. Six2 is a transcription factor that is specifically expressed in the metanephric mesenchyme.

Subsequently, when the diphtheria toxin is brought into contact with the metanephros of the above-mentioned mouse, cells specifically in the metanephric mesenchyme are killed, and thus the metanephric mesenchyme can be tissue-specifically removed. That is, the kidney development niche can be emptied.

The contact with the diphtheria toxin may be carried out by injecting the diphtheria toxin into a parent mouse or a fetus of a mouse. Alternatively, the contact may be carried out by extracting the metanephros from a fetus of a mouse, organ-culturing the extracted metanephros, adding the diphtheria toxin to a medium, and the like. Alternatively, the contact may be carried out by transplanting the extracted metanephros into a para-aortic region or greater omentum of a patient or affected animal, locally administering the diphtheria toxin into a body of the patient or affected animal, and the like. In the present specification, examples of the patient or affected animal include a human and non-human animal. In the present specification, examples of the non-human animal include cats, dogs, horses (especially race horses), monkeys, cows, sheep, pigs, goats, rabbits, hamsters, guinea pigs, rats, mice, and the like.

The above-described method is one of methods of tissue-specifically removing the metanephric mesenchyme, and can be applied to not only mice but also any non-human animal. In addition, the same method is not limited to the kidney, and can be applied to various other organs. The method can be applied to, for example, removal of pancreatic precursor cells in the pancreas, removal of hepatic precursor cells in the liver, removal of adrenal precursor cells in the adrenal gland, and the like.

### (Step (ii))

Subsequently, in the present step, organ precursor cells which is allogeneic or xenogeneic to the non-human animal are transplanted into the above-described organ. The step (i) and step (ii) may be carried out in parallel. That is, a time of emptying the niche and a time of transplanting the organ precursor cells may overlap. For example, the addition of the removing medicine and the transplantation of the organ precursor cells may be carried out at the same time.

Examples of the organ precursor cells include organ precursor cells differentiation-induced from pluripotent stem cells such as mesenchymal stem cells (MSCs), iPS cells, and ES cells derived from mammals. The organ precursor cells may be cells derived from the patient or affected animal or allogeneic cells whose rejection reaction is suppressed in the patient or affected animal.

It is preferable that the organ precursor cells be cells derived from the patient or affected animal that is a subject of the transplantation of the organ after production. In a case where the subject of the transplantation is a human, examples of the organ precursor cells differentiation-induced from mesenchymal stem cells sorted from human bone marrow, adipose tissue, bloodshed, or umbilical cord blood. The organ precursor cells may be organ precursor cells differentiation-induced from mesenchymal stem cells sorted from bone marrow, bloodshed, or umbilical cord blood of a patient himself. A sorting method may be a method according to general surgical medical techniques. It is preferable that the sorted cells be cultivated for 2 to 5 times by selecting optimum conditions. In addition, the cultivation may be carried out by using a medium kit exclusive for human mesenchymal stem cells manufactured by Cambrex BioScience, or the like for the purpose of continuing the cultivation while suppressing the transformation of the mesenchymal stem cells.

If desired, a desired gene may be introduced into the organ precursor cells using adenovirus, retrovirus, or the like. For example, in a case of kidney precursor cells, the gene may be introduced so as to express glial cell line-derived neurotrophic factor-GDNF for the purpose of assisting kidney formation. This is because the mesenchymal tissue immediately before the kidney is formed becomes to express GDNF, and the ureteric bud expressing c-ret which is a receptor thereof is drawn, thereby completing a first important step of the kidney development.

As a method of transplantation, for example, the organ precursor cells may be injected into the organ using an injection needle or the like. The number of organ precursor cells to be transplanted varies depending on the subject of the organ, but may be about 1 × 10³ to 1 × 10⁶, for example. Because the transplantation of the organ precursor cells can be performed in vitro, skill for the operation is not required, and the operation is easily performed.

The organ precursor cells may be human cells. In this case, the organ to be produced is composed of human cells transplanted into the organ development niche. This organ can be transplanted into a patient so as to function. The organ precursor cells are more preferably cells in which the rejection reaction with cells of the patient himself or the patient is suppressed.

The organ precursor cells may be non-human animal cells. Examples of non-human animals include animals described above as the affected animal. In this case, the organ to be produced is composed of non-human animal cells transplanted into the organ development niche. This organ can be transplanted into the non-human animal that is the affected animal so as to function. The organ precursor cells are more preferably cells in which the rejection reaction with cells of the affected animal or the affected animal is suppressed.

The organ precursor cells may be cat cells. In this case, the organ to be produced is composed of cat cells transplanted into the organ development niche. This organ can be transplanted into a cat that is an affected animal so as to function. The organ precursor cells are more preferably cells in which the rejection reaction with cells of the affected animal or the affected animal is suppressed.

### (Step (iii))

Subsequently, in the present step, the development of the organ after transplanting the organ precursor cells progresses. As a result, the transplanted organ precursor cells are differentiated and matured, thereby forming a part of the organ. In a case where the organ precursor cells are transplanted without extracting the organ from the embryo, the progression of the organ can be carried out by returning the embryo back to the uterus of the parent animal, performing whole embryo culture, and the like. Alternatively, in a case where the organ is extracted to transplant the organ precursor cells, the progression of the development of the organ can be carried out by continuing an organ culture of the organ, and the like. Alternatively, in a case where the organ is transplanted into an appropriate region in the body of the patient or affected animal, and the organ precursor cells are transplanted into the body of the patient or affected animal, the progression of the organ can be carried out by growing the organ as it is. Examples of the appropriate region include, in the case of the metanephros, a para-aortic region or greater omentum of the patient or affected animal, and the like.

As a result, as will be described later in Examples using the kidney as an example, the transplanted organ precursor cells take over the developmental program of the host, autonomously advance complicated differentiation induction while interacting with the remaining tissues (tissues other than the first portion, which is an ureteric bud in the Example), and thereby forming complex tissues (glomeruli and kidney tubules in the case of the kidney). In addition, in order to completely remove the organ precursor cells constituting the first portion on the host side, the tissues to be regenerated are composed only of cells derived from substantially the transplanted organ precursor cells.

Similarly, even in a case where the organ is an organ other than the kidney, the transplanted organ precursor cells take over the developmental program of the host, autonomously advance complicated differentiation induction while interacting with the remaining organ tissues derived from the host, and thereby forming a functional organ. Also in this case, the tissues to be regenerated substantially consist only of cells derived from the transplanted organ precursor cells.

The term "substantially" means that in a case where the organ is produced using the organ development niche of the non-human animal, mixing of cells other than the transplanted organ precursor cells is not excluded. In other words, it is preferable that 70% by mass or more of the tissues to be regenerated be composed of the transplanted organ precursor cells, it is more preferable that 80% by mass or more of the tissues to be regenerated be composed of the transplanted organ precursor cells, it is even more preferable that 90% by mass or more of the tissues to be regenerated be composed of the transplanted organ precursor cells, it is still more preferable that 95% by mass or more of the tissues to be regenerated be composed of the transplanted organ precursor cells, and it is particularly preferable that 99% by mass or more of the tissues to be regenerated be composed of the transplanted organ precursor cells.

The organ production method of the present embodiment may further include a step (iv) of tissue-specifically removing a portion different from the first portion of the organ partway through the development thereof; and a step (v) of transplanting, into the organ, an organ precursor cell which is allogeneic or xenogeneic to the non-human animal.

The portion different from the first portion of the organ means a portion different from the first portion described above among a plurality of tissues in which a development program progresses while interacting in the development of the organ. As a result, not only the first portion but also a portion different from the first portion can be replaced with cells derived from the transplanted organ precursor cells. As a result, it is possible to produce the organ substantially consisting only of the cells derived from the transplanted organ precursor cells.

In a case where the portion different from the first portion of the organ includes, for example, a second portion, a third portion, a fourth portion, and the like, it is possible to produce the organ in which all of the tissues consisting the organ are the cells derived from the transplanted organ precursor cells by repeating the same step. Hereinafter, the steps (iv) and (v) will be described.

### (Step (iv))

In the present step, the portion different from the first portion of the organ partway through the development thereof is tissue-specifically removed. The tissue-specific removal of the portion different from the first portion is not particularly limited as long as the portion different from the first portion can be tissue-specifically removed, and can be performed by using, for example, a genetic recombination technique.

A case of the kidney will be described more specifically using examples. As an example, the first portion is referred to as a metanephric mesenchyme, and the portion different from the first portion is referred to as an ureteric bud. In order to tissue-specifically removing the ureteric bud of the kidney partway through the development thereof, for example, it is possible to use a mouse obtained by further introducing a gene encoding a Cre-ER protein into downstream of an ureteric bud-specific promoter in the mouse obtained by crossing the iDTR mouse and the Six2-Cre mouse described above. Examples of the ureteric bud-specific promoter include a promoter of cytokeratin 8, a promoter of HoxB7, and the like.

Examples of such a mouse include an iDTR mouse having a genotype of Six2-Cre^{tg/wt} cytokeratin 8-Cre-ER^{tg/wt}, an iDTR mouse having a genotype of Six2-Cre^{tg/wt}HoxB7-Cre-ER^{tg/wt}, and the like. The term "tg" represents that the type is transgenic, and "wt" represents a wild type.

The Cre-ER protein is a fusion protein of a Cre recombinase and a mutant estrogen receptor. The above-described mouse expresses Cre-ER in a promoter-dependent manner with respect to cytokeratin 8 or HoxB7, which is a marker for the ureteric bud.

Although the Cre-ER protein is generally present in the cytoplasm, the Cre-ER protein is translocated into the nucleus by binding with tamoxifen, which is an estrogen derivative, and recombines against the loxP sequence. By utilizing the above description, It is possible to tamoxifen-dependently adjust a working time of a Cre-loxP system. Therefore, even if Cre-ER and Cre are expressed at the same time, it is possible to control the expression of Cre-ER activity depending on whether tamoxifen is administered or not. Cre-ERT, Cre-ERT2, or the like, which is a modified form of Cre-ER, may be used instead of Cre-ER.

The mouse described above expresses the diphtheria toxin receptor specifically in the metanephric mesenchyme. In this case, it is preferable to first perform the steps (i), (ii), and (iii) described above. Specifically, the metanephric mesenchyme (first portion) is removed by bringing the diphtheria toxin into contact with the metanephros of the mouse embryo. Subsequently, the kidney precursor cells are transplanted into the metanephros by the above-described method so that the development of the metanephros progresses. As a result, the metanephric mesenchyme is regenerated by the transplanted kidney precursor cells.

Subsequently, when tamoxifen is brought into contact with this metanephros, the diphtheria toxin receptor is expressed specifically in the ureteric bud. In this case, by bringing the diphtheria toxin into contact with this metanephros, the ureteric bud (the portion different from the first portion) can be tissue-specifically removed. That is, the kidney development niche can be emptied.

In the above example of the mouse, the gene encoding the Cre protein is introduced into downstream of the metanephric mesenchyme-specific promoter, and the gene encoding the Cre-ER protein is introduced into downstream of the ureteric bud-specific promoter. However, the gene encoding the Cre-ER protein may be introduced into downstream of the metanephric mesenchyme-specific promoter, and the gene encoding the Cre protein may be introduced into downstream of the ureteric bud-specific promoter. In addition, Cre-ERT, Cre-ERT2, or the like, which is a modified form of Cre-ER, may be used instead of Cre-ER.

For example, a mouse obtained by further introducing the gene encoding the Cre protein into downstream of the ureteric bud-specific promoter in the mouse obtained by crossing the iDTR mouse with the mouse in which the gene encoding the Cre-ER protein is introduced into downstream of the metanephric mesenchyme-specific promoter, may be used.

More specific examples of the mouse include an iDTR mouse having a genotype of Six2-Cre-ER^{tg/wt}HoxB7-Cre^{tg/wt}, and the like. The terms "tg" and "wt" have the same meanings as those described above. In this example, the first portion is referred to as the ureteric bud, and the portion different from the first portion is referred to as the metanephric mesenchyme.

This mouse expresses Cre-ER specifically in the metanephric mesenchyme. In addition, the diphtheria toxin receptor is specifically expressed in the ureteric bud. Therefore, in the case of using this mouse, the steps (iv) and (v) are carried out before carrying out the steps (i) to (iii). Specifically, by bringing the diphtheria toxin into contact with the metanephros of the mouse embryo, the ureteric bud (first portion) can be removed. Subsequently, the kidney precursor cells are transplanted into the metanephros by the above-described method so that the development of the metanephros progresses. As a result, the ureteric bud is regenerated by the transplanted kidney precursor cells of a human.

Subsequently, when tamoxifen is brought into contact with this metanephros, the diphtheria toxin receptor is expressed specifically in the metanephric mesenchyme (the portion different from the first portion) at this time. In this case, by bringing the diphtheria toxin into contact with this metanephros, the metanephric mesenchyme (the portion different from the first portion) can be tissue-specifically removed at this time. That is, the kidney development niche can be emptied.

The above-described method is one of methods of tissue-specifically removing the portion different from the first portion, and can be applied to not only mice but also any non-human animal.

### (Step (v))

Subsequently, in the present step, organ precursor cells which is allogeneic or xenogeneic to the non-human animal are transplanted into the above-described organ. The step (iv) and step (v) may be carried out in parallel. That is, a time of emptying the niche and a time of transplanting the organ precursor cells may overlap. For example, the addition of the removing medicine and the transplantation of the organ precursor cells may be carried out at the same time.

Subsequently, the above-described step (iii) is carried out so that the development of the organ (metanephros in the above-described example) transplanted with the organ precursor cells progresses. As a result, the transplanted organ precursor cells are differentiated and matured, thereby forming a part of the organ.

As the organ precursor cells, cells that are the same as those described above can be used. In a case where the organ precursor cells have the same origin as that of the organ precursor cells in regeneration of the first portion described above, not only the first portion but also the portion different from the first portion are composed of cells of the same origin in the organ to be produced.

Therefore, if the organ precursor cells are cells of the patient or affected animal or cells in which the rejection reaction with the patient or affected animal is suppressed, it is possible to produce the kidney with less rejection reaction in a case of transplanting these organ precursor cells into the patient or affected animal.

In the organ production method of the present embodiment, any of the removal of the first portion and the removal of the portion different from the first portion may be performed first. In a case of removing the portion different from the first portion first, the above-described Cre-loxP system may be appropriately modified. In addition, in the case where the portion different from the first portion of the organ includes the second portion, the third portion, the fourth portion, and the like, it is possible to produce the organ in which all portions are derived from the transplanted organ precursor cells by repeating the same step.

In the organ production method of the present embodiment, a system for killing cells can be used without particular limitation as long as the system can be applied to a target non-human animal species and can be operated in a tissue-specific manner or in a time-specific manner.

Therefore, a configuration in which the cells are killed by a system other than the diphtheria toxin receptor may be adopted. Examples thereof include a system that expresses a diphteria toxin A subunit (DT-A) at downstream of a tissue-specific promoter in the Cre recombinase activity-dependent manner. This system can be applied to non-human animals which are originally sensitive to the diphtheria toxin. Examples of such a non-human animal include a pig.

Alternatively, examples thereof include a system that expresses a herpes simplex virus-derived thymidine kinase gene (HSV-TK) which induces apoptosis by administration of ganciclovir, at downstream of the tissue-specific promoter. In cells expressing HSV-TK, cell death is induced under administration of ganciclovir.

Alternatively, a system that induces apoptosis by dimerizing Caspase-3, Caspase-8, Caspase-9, and the like by administration of AP20187 may be used.

The organ production method of the present embodiment is not limited to the method described above as long as the removal of the first portion and removal of the portion different from the first portion can be selectively performed, and it is possible to use various genetic recombination systems and a combination of genetic recombination systems.

### [Organ]

In one embodiment, the present invention provides an organ produced by the production method described above. The organ of the present embodiment is produced from desired organ precursor cells by utilizing the organ development niche and the developmental program of a host non-human animal. Therefore, the kidney can be transplanted into the patient or affected animal so as to function.

As described above, at least a part of the organ of the present embodiment is composed of the transplanted kidney precursor cells. In particular, in the case where the entire organ is an organ replaced with the transplanted organ precursor cells, merely by specifying the cells constituting the organ, it is difficult to specify whether an organ is the organ produced by the above-described production method, or is the organ of the patient or affected animal from which the transplanted organ precursor cells had been prepared.

The organ includes cells derived from a non-human animal; and cells which is allogeneic or xenogeneic to the non-human animal, in which a proportion of the cells derived from the non-human animal which is allogeneic or xenogeneic to the non-human animal is 70% by mass or more, more preferably 80% by mass or more, even more preferably 90% by mass or more, still more preferably 95% by mass or more, and particularly preferably 99% by mass or more.

Examples of the non-human animals include pigs, mice, cats, and the like as described above. In addition, examples of the cells which is allogeneic or xenogeneic to these non-human animals include human cells and non-human animal cells. Examples of the non-human animal cells include cells derived from a non-human animal described above as an affected animal, and a cat cell may be used for example. Such an organ can be produced by the above-described organ production method.

The organ of the present embodiment may be formed by replacing the first portion with the transplanted organ precursor cells according to the above-described method, for example. In this case, the tissue derived from the portion other than the first portion is derived from a non-human animal host.

Alternatively, the organ of the present embodiment may be formed by all portions composing the organ with the transplanted organ precursor cells according to the above-described method. In this case, the entire organ is substantially derived from the transplanted organ precursor cells. However, cells derived from a host non-human animal may remain in the formed organ in some cases.

The organ of the present embodiment can be extracted from a non-human animal, stored, and distributed. The organ may be frozen for storage and distribution. Alternatively, the organ of the present embodiment can be distributed in a form of a non-human animal having the organ of the present embodiment.

### [Genetically Modified Non-Human Animal]

In one embodiment, the present invention provides a genetically modified non-human animal which is capable of tissue-specifically removing the first portion of the organ partway through the development thereof. The genetically modified non-human animal of the present embodiment is for producing the organ described above. The genetically modified non-human animal may be, for example, a genetically modified mouse or a genetically modified pig. In addition, a genetic modification method is not particularly limited, and may be a method using ES cells or a method using genome editing.

More specific examples of the genetically modified non-human animal of the present embodiment include, when using the kidney for example as the organ, a genetically modified non-human animal that has a construct expressing a diphtheria toxin receptor (DTR) in a Cre recombinase activity-dependent manner as described above, and a construct in which a Cre recombinase gene is connected downstream of the metanephric mesenchyme-specific promoter. This system can be applied to non-human animals which are not originally sensitive to the diphtheria toxin. Examples of such a non-human animal include a mouse.

Examples of the metanephric mesenchyme-specific promoter include a promoter of Six2 which is a transcription factor, and the like. This genetically modified non-human animal expresses Cre recombinase specifically in the metanephric mesenchyme. As a result, the diphtheria toxin receptor is expressed specifically in the metanephric mesenchyme. In this case, when the diphtheria toxin is brought into contact with the metanephros, the metanephric mesenchyme can be specifically killed and removed.

As Cre recombinase, Cre-ER capable of translocating Cre recombinase into the nucleus in the presence of tamoxifen, and the like may be used. In this case, a complex of an estrogen receptor and a Cre protein can be translocated into the nucleus only in a case where tamoxifen is administered to express Cre recombinase activity, and therefore the diphtheria toxin receptor can be expressed.

Examples of the genetically modified non-human animal of the present embodiment further include a genetically modified non-human animal that has a construct expressing diphtheria toxin (diphteria toxin A subunit: DT-A) in a Cre recombinase activity-dependent manner, and a construct in which a Cre recombinase gene is connected downstream of the metanephric mesenchyme-specific promoter. This system can be applied to non-human animals which are originally sensitive to the diphtheria toxin. Examples of such a non-human animal include a pig.

Examples of the metanephric mesenchyme-specific promoter include a promoter of Six2 which is a transcription factor, and the like. This genetically modified non-human animal expresses Cre recombinase specifically in the metanephric mesenchyme. As a result, diphtheria toxin can be specifically expressed in the metanephric mesenchyme so as to be killed and removed.

As Cre recombinase, Cre-ER capable of translocating Cre recombinase into the nucleus in the presence of tamoxifen, and the like may be used. In this case, Cre recombinase can be translocated into the nucleus to express the diphtheria toxin only in a case where tamoxifen is administered. In addition, Cre-ERT, Cre-ERT2, or the like, which is a modified form of Cre-ER, may be used instead of Cre-ER.

The non-human animal of the present embodiment may further be genetically modified so that the portion different from the first portion of the organ can be tissue-specifically removed. More specifically, when using the kidney for example as the organ, the non-human animal of the present embodiment may further be genetically modified so that not only the above-described metanephric mesenchyme but also the ureteric bud of the metanephros can be tissue-specifically removed. Examples thereof include a genetically modified non-human animal which further has a construct in which the Cre recombinase gene is connected downstream of the ureteric bud-specific promoter, in addition to the construct expressing the diphtheria toxin receptor (DTR) in the Cre recombinase activity-dependent manner, and the construct in which the Cre recombinase gene is connected downstream of the metanephric mesenchyme-specific promoter. This system can be applied to non-human animals which are not originally sensitive to the diphtheria toxin. Examples of such a non-human animal include a mouse.

Examples of the ureteric bud-specific promoter include a promoter of cytokeratin 8, a promoter of HoxB7, and the like. This genetically modified non-human animal expresses Cre recombinase specifically in the ureteric bud. As a result, the diphtheria toxin receptor is specifically expressed in the ureteric bud. In this case, when the diphtheria toxin is brought into contact with the metanephros, the ureteric bud can be specifically killed and removed. As the Cre recombinase, Cre-ER, Cre-ERT, Cre-ERT2, and the like may be used.

In addition, in the genetically modified non-human animal of the present embodiment, a system for killing cells can be used without particular limitation as long as the system can be applied to a target non-human animal species and can be operated in a tissue-specific manner or in a time-specific manner.

Therefore, a configuration in which the cells are killed by a system other than the diphtheria toxin or diphtheria toxin receptor may be adopted. Examples thereof include a system that expresses a herpes simplex virus-derived thymidine kinase gene (HSV-TK) which induces apoptosis by administration of ganciclovir, at downstream of the tissue-specific promoter. In cells expressing HSV-TK, cell death is induced under administration of ganciclovir.

Alternatively, a system that induces apoptosis by dimerizing Caspase-3, Caspase-8, Caspase-9, and the like by administration of AP20187 may be used.

The genetically modified non-human animal of the present embodiment is not limited to the method described above as long as the metanephric mesenchyme or the ureteric bud of the metanephros can be tissue-specifically removed, and may have various genetic recombination systems and a combination of genetic recombination systems.

The genetically modified non-human animal of the present embodiment is not limited to the method described above as long as the first portion of the organ, and, if necessary, the portion different from the first portion can be tissue-specifically removed, and may have various genetic recombination systems and a combination of genetic recombination systems. In addition, the organ is not limited to the kidney, and may be, for example, heart, liver, pancreas, adrenal gland, intestinal tract, and the like.

### [Kit for Organ Production]

In one embodiment, the present invention provides a kit for organ production including an organ of a non-human animal partway through the development thereof; a medicine which tissue-specifically removes a first portion of the organ; and an organ precursor cell which is allogeneic or xenogeneic to the non-human animal.

By carrying out the above-mentioned organ production method using the kit of the present embodiment, the organ can be produced. Examples of the non-human animals include pigs, mice, cats, and the like as described above. The organ of the non-human animal may be provided in a form of an embryo or may be provided in a form of a parent animal including a fetus. The organ is not particularly limited, and examples thereof include kidney, heart, liver, pancreas, adrenal gland, intestinal tract, and the like.

In addition, examples of a medicine which tissue-specifically removes the first portion of the organ include the above-mentioned diphtheria toxin, and the like. In this case, examples of a non-human animal include a non-human animal that expresses the diphtheria toxin receptor specifically in the first portion (metanephric mesenchyme in the above-described example) by the Cre-loxP system or the like.

Alternatively, various conditional knockout systems other than those described above can also be used as long as the first portion of the organ can be tissue-specifically removed thereby. As the medicine for tissue-specifically removing the first portion of the organ, diphtheria toxin (iDTR system), tamoxifen (Cre-ERT2 system), tacrolimus (Mos-iCsp3 system), doxycycline, tetracycline (Tet-On/off system), ganciclovir (herpes virus-derived thymidine kinase gene system), and the like can be used depending on a system to be used.

These conditional knockout systems can be produced using genome editing techniques and the like, and can tissue-specifically remove cells in the organ development niche of the non-human animal. In addition, a time at which cells are removed can be controlled by administering the medicine. By using these systems, it is possible to spatiotemporal-specifically control the removal of target cells and construct a living scaffold suitable for the organ regeneration.

A method for administering the medicine that tissue-specifically removes the first portion of the organ is not particularly limited, and may be appropriately selected according to the medicine to be used. Examples thereof include addition to a medium such as organ culture in vitro, local administration in vivo, intraperitoneal administration, intravenous injection, oral administration, and the like.

In addition, examples of the organ precursor cells which is allogeneic or xenogeneic to the non-human animal include the organ precursor cells derived from the patient or affected animal, and the like. Examples of the affected animal include those mentioned above. Examples of the organ precursor cells include organ precursor cells differentiation-induced from iPS cells, mesenchymal stem cells (MSCs), and the like which are derived from the patient or affected animal; organ precursor cells differentiation-induced from pluripotent stem cells such as allogeneic iPS cells or ES cells, in which the rejection reaction in the patient or affected animal is suppressed; and the like. The allogeneic iPS cells described above can be obtained from, for example, an iPS cell bank.

The organ production may be carried out outside the body of the patient or affected animal, and then the organ may be transplanted into the appropriate region of the body of the patient or affected animal. Examples of the appropriate region include, in the case of the kidney, a para-aortic region or greater omentum, and the like.

Alternatively, the organ production may be performed in the body of the patient or affected animal. More specifically, the organ may be produced by, first, transplanting the organ of the non-human animal partway through the development thereof into an appropriate region (for example, a para-aortic region or greater omentum, and the like) of the patient or affected animal, emptying the organ development niche in the body of the patient or affected animal, and transplanting the organ precursor cells. This method is advantageous in that a vascular system drawn into the regenerated organ becomes a blood vessel of the patient or affected animal, and that a functional organ can be constructed.

The kit of the present embodiment may further include a medicine that tissue-specifically removes the portion different from the first portion of the organ. Accordingly, it is possible to produce the organ derived from the organ precursor cells transplanted with not only tissues derived from the first portion but also tissues derived from the portion different from the first portion.

Examples of the medicine that tissue-specifically removes the portion different from the first portion include a combination of tamoxifen and diphtheria toxin as described above, and the like. In this case, examples of a non-human animal include a non-human animal that expresses the diphtheria toxin receptor specifically in the first portion by the Cre-loxP system or the like, and also expresses the diphtheria toxin receptor specifically in the portion different from the first portion by administration of tamoxifen.

Alternatively, any of the various conditional knockout systems described above can be used to remove the portion different from the first portion. In this case, the medicine that tissue-specifically removes the portion different from the first portion becomes an appropriate medicine according to an adopted system.

By combining the above-described various conditional knockout systems, it is possible to remove a plurality of target tissues at any timing. For example, after tissue-specifically removing the portion different from the first portion by using tamoxifen, the first portion can be tissue-specifically removed by using tacrolimus. As a result, almost all tissues composing the organ can be tissues derived from the transplanted organ precursor cells.

### [Medical Drug for Organ Regeneration]

In one embodiment, the present invention provides a medical drug for organ regeneration, including an organ of a non-human animal partway through the development thereof; a medicine that tissue-specifically removes a first portion of the organ; and an organ precursor cell.

According to the medical drug of the present embodiment, it is possible to perform the regeneration of the organ within the body of the patient or affected animal as described above. Examples of the affected animal include those mentioned above. The medical drug of the present embodiment can be used as follows. First, an organ of the non-human animal partway through the development thereof is transplanted into the appropriate region (for example, a para-aortic region or greater omentum, and the like) of the patient or affected animal. As the organ of the non-human animal, the same metanephros as in the above-described kit for organ production can be used.

Subsequently, a medicine that tissue-specifically removes the first portion of the organ is administered to the patient or affected animal. As the medicine, the same medicine as in the above-described kit for organ production can be used. It is preferable that the administration of the medicine be appropriately carried out by local administration, intraperitoneal administration, intravenous injection, oral administration, and the like depending on the medicine to be used.

Subsequently, the organ precursor cells are transplanted. Examples of the organ precursor cells include organ precursor cells differentiation-induced from iPS cells, mesenchymal stem cells (MSCs), and the like which are derived from the patient or affected animal; organ precursor cells differentiation-induced from allogeneic iPS cells or ES cells, in which the rejection reaction in the patient or affected animal is suppressed; and the like. Thereafter, the development of the transplanted organ progresses, thereby producing (regenerating) the organ composed of the organ precursor cells transplanted into the body of the patient or affected animal.

This medical drug of the present embodiment is advantageous in that a vascular system drawn into the regenerated organ becomes a blood vessel of the patient or affected animal, and that a functional organ can be constructed.

The medical drug for organ regeneration of the present embodiment may further include a medicine which tissue-specifically removes the portion different from the first portion of the organ. Accordingly, it is possible to regenerate the organ derived from the organ precursor cells transplanted with not only tissues derived from the first portion but also tissues derived from the portion different from the first portion. As the medicine which tissue-specifically removes the portion different from the first portion of the organ, the same medicine as in the above-described kit for organ production can be used.

### [Other Embodiments]

In one embodiment, the present invention provides a method for treating an organ disease, including a step of transplanting the above-described organ into the body of the patient or affected animal. Examples of the affected animal include the same affected animal as mentioned above.

In one embodiment, the present invention provides a method for treating an organ disease, including a step (a) of transplanting the organ of the non-human animal partway through the development thereof into the body of the patient or affected animal; a step (b) of tissue-specifically removing the first portion of the organ; and a step (c) of transplanting the organ precursor cells into the organ, which is a step in which the transplanted organ precursor cells are differentiated and matured to form a part of the organ. Examples of the affected animal include the same affected animal as mentioned above.

In the method of the present embodiment, the production (regeneration) of the organ is performed in the body of the patient or affected animal. The step (b) and the step (c) may be carried out in parallel. That is, a time of emptying the niche and a time of transplanting the organ precursor cells may overlap. For example, the addition of the removing medicine and the transplantation of the organ precursor cells may be carried out at the same time.
Examples of the organ precursor cells include the same kidney precursor cells as those for the above-described medical drug for organ regeneration.

The treatment method of the present embodiment may further include a step (b') of tissue-specifically removing the portion different from the first portion. As a result, not only the first portion but also a portion different from the first portion can be replaced with cells derived from the transplanted organ precursor cells. As a result, it is also possible to produce the organ substantially consisting only of the cells derived from the transplanted organ precursor cells.

In a case of carrying out the step (b'), any of the step (b) and the step (b') may be carried out first, but after carrying out either one step thereof, it is preferable to provide a period of, for example, 4 to 7 days before carrying out the other step. Between the steps, transplanted organ precursor cells progress the development program to be differentiated. The above-mentioned period is appropriately adjusted according to the organ to be produced or tissue.

The step (b) and step (c), or the step (b') and step (c) may be carried out in parallel. That is, a time of emptying the niche and a time of transplanting the organ precursor cells may overlap. For example, the addition of the removing medicine and the transplantation of the organ precursor cells may be carried out at the same time.

For example, the step (b) and step (c) may be carried out at the same time first, and after a predetermined period, the step (b') and step (c) may be carried out at the same time. Alternatively, the step (b') and step (c) may be carried out at the same time first, and after a predetermined period, the step (b) and step (c) may be carried out at the same time.

### Examples

Next, the present invention will be described in more detail by showing experimental examples, but the present invention is not limited to the following experimental examples.

### [Experimental Example 1]

### (Tissue-Specific Removal of Metanephric Mesenchyme in Mouse Embryo)

The iDTR mouse expressing the diphtheria toxin receptor (DTR) in the Cre recombinase activity-dependent manner was crossed with the Six2-Cre mouse in which the Cre recombinase gene was introduced into downstream of a promoter of Six2. Six2 is a transcription factor that is specifically expressed in the metanephric mesenchyme.

Subsequently, a mouse that expressed the diphtheria toxin receptor specifically in the metanephric mesenchyme was selected from an F₁ mouse on embryonic day 13, and the metanephros was extracted. The extracted metanephros was organ-cultured according to a standard method, and diphtheria toxin was added to the medium. As a result, Six2-positive kidney precursor cells of the metanephric mesenchyme, in which the diphtheria toxin receptor was expressed were killed. As a result, the metanephric mesenchyme was tissue-specifically removed.

### [Experimental Example 2]

### (Transplantation of Kidney Precursor Cells)

The kidney precursor cells derived from a wild-type mouse were prepared from the metanephros by a standard method and dissociated into single cells using a cell dissociation reagent. Subsequently, 1 × 10⁵ kidney precursor cells were transplanted into metanephros tissue from which the metanephric mesenchyme was tissue-specifically removed, which was prepared in Experimental Example 1. Thereafter, the organ culture of the metanephros tissue was continued.

### [Experimental Example 3]

### (Immunostaining 1 of Kidney Tissue)

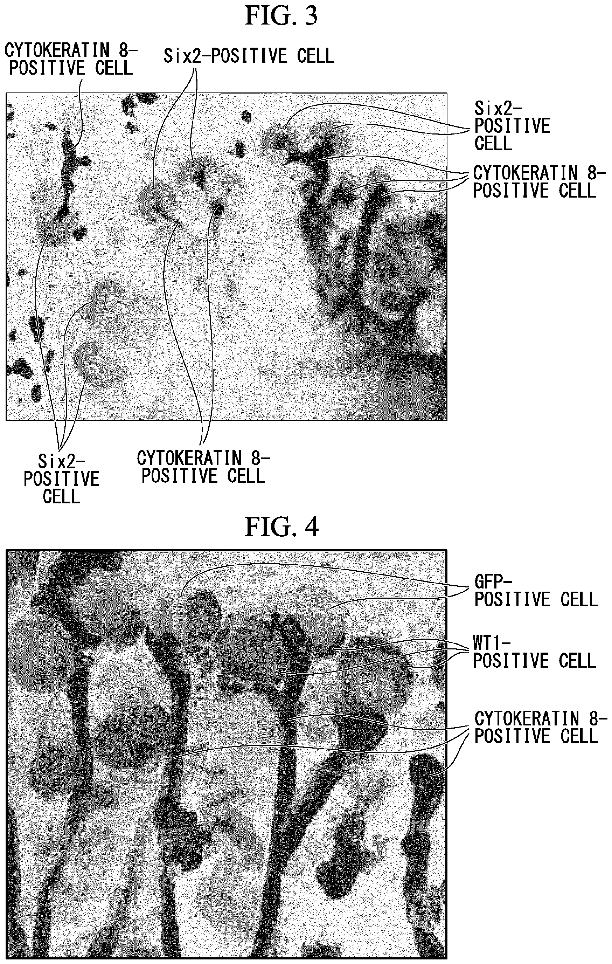

The metanephros tissue of Experimental Example 2 was fixed with 4% paraformaldehyde after being cultured for 5 days from the transplantation of the kidney precursor cells, and therefore a tissue section sample was prepared. Subsequently, Six2 which is a marker of the metanephric mesenchyme and cytokeratin 8 which is a marker of the ureteric bud were immunostained, respectively. FIG. 3 is a fluorescence microscopy photograph showing the result of immunostaining. The magnification is 100 times. In FIG. 3, Six 2-positive cells are derived from the transplanted kidney precursor cells. In addition, cytokeratin 8-positive cells are derived from the host animal (F₁ mouse of Experimental Example 1).

As a result, it was confirmed that, using the ureteric bud derived from the F₁ mouse of Experimental Example 1 as a scaffold, the kidney precursor cells transplanted in Experimental Example 2 took over the development program of the host, autonomously advanced complicated differentiation induction, and thus formed the metanephric mesenchyme. This result shows that the cells constituting the metanephric mesenchyme can be replaced with the cells derived from the transplanted kidney precursor cells by the above-described method.

### [Experimental Example 4]

### (Immunostaining 2 of Kidney Tissue)

The kidney precursor cells derived from a transgenic mouse (GFP tg mouse) into which a green fluorescent protein (GFP) gene was introduced were prepared from the metanephros by a standard method and dissociated into single cells using a cell dissociation reagent. Subsequently, 1 × 10⁵ kidney precursor cells were transplanted into metanephros tissue from which the metanephric mesenchyme was tissue-specifically removed, which was prepared in the same manner as in Experimental Example 1. Thereafter, the organ culture of the metanephros tissue was continued.

Subsequently, the metanephros tissue was fixed with 4% paraformaldehyde after being cultured for 7 days from the transplantation of the kidney precursor cells, and therefore a tissue section sample was prepared. Subsequently, cytokeratin 8, which is a marker for the ureteric bud, and Wilms tumor suppressor protein-1 (WT1), which is a marker of glomerulus, were immunostained, respectively. Subsequently, fluorescence of GFP expressing the transplanted cells, and immunostained cytokeratin 8 and WT1 was observed with a fluorescence microscope. FIG. 4 is a fluorescence microscopy photograph showing the result of immunostaining. The magnification is 200 times. In FIG. 4, GFP-positive cells and WT1-positive cells are derived from the transplanted kidney precursor cells. In addition, cytokeratin 8-positive cells are derived from the host animal (F₁ mouse of Experimental Example 1).

As a result, it was confirmed that the transplanted kidney precursor cells derived from the GFP tg mice formed the glomeruli and kidney tubules. This result shows that it is possible to form functional kidney tissue from the kidney precursor cells by the method described above.

### [Experimental Example 5]

### (Regeneration of Kidney in Living Body of Mouse)

In the para-aortic region of the wild-type mouse, the cloaca and the metanephros tissue-specifically expressing the diphtheria toxin receptor in the metanephros tissue, which are obtained by crossing the iDTR mouse with Six2-Cre mouse, were transplanted.

Subsequently, by administering the diphtheria toxin, the metanephric mesenchyme of the metanephros was removed, and 1×10⁵ kidney precursor cells derived from the GFP tg mouse were transplanted. Subsequently, a mouse transplanted with the metanephros was raised for 7 days.

Subsequently, an abdominal cavity of the above-mentioned mouse was opened to observe the kidney (regenerated kidney) formed from the transplanted metanephros.

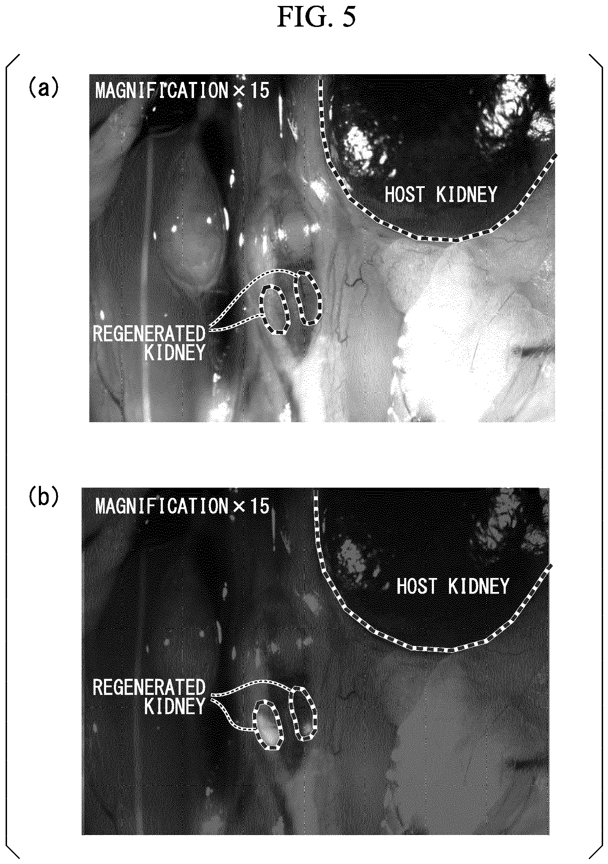

(a) of FIG. 5 is a photograph of a region where regenerated kidneys exist. The magnification is 15 times. The upper right shows the kidney of the wild-type mouse (host). The regenerated kidneys exist in a region surrounded by a dotted line near the center.

(b) of FIG. 5 is a photograph showing the observation result of the fluorescence of GFP in the same field of view as (a) of FIG. 5. The magnification is 15 times. As a result, fluorescence of GFP derived from transplanted kidney precursor cells was observed. This result indicates that the kidney regeneration was possible in the body of the mouse.

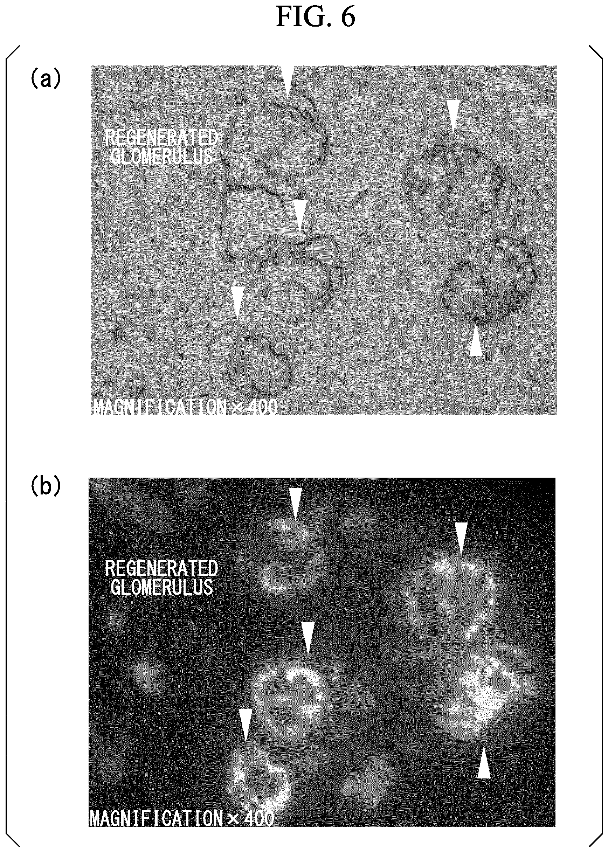

(a) of FIG. 6 is an optical micrograph of a tissue section sample prepared by fixing the above regenerated kidneys with 4% paraformaldehyde. The magnification is 400 times. In (a) of FIG. 6, the regenerated glomeruli were observed at a portion indicated by an arrowhead.

(b) of FIG. 6 is a fluorescence micrograph showing the observation result of the fluorescence of GFP in the same field of view as (a) of FIG. 6. The magnification is 400 times. In (b) of FIG. 6, fluorescence of GFP was observed at a portion indicated by an arrowhead. This result indicates that the regenerated glomeruli are derived from the transplanted kidney precursor cells.

Based on the above results, it became clear that the kidney regeneration can be performed in vivo.

### [Experimental Example 6]

### (Replacement of Both Metanephric Mesenchyme and Ureteric Bud of Mouse Embryo)

### «Tissue-Specific Removal of Ureteric Bud»

In the present experimental example, the iDTR mouse having a genotype of Six2-Cre-ER^{tg/wt}HoxB7-Cre^{tg/wt} was used. First, in the same manner as in Experimental Example 1, the metanephros was extracted from the mouse embryo and subjected to organ-culture. Subsequently, diphtheria toxin was added to the medium to tissue-specifically remove the ureteric bud.

### <<Transplantation of Kidney Precursor Cells>>

Subsequently, in the same manner as in Experimental Example 2, 1×10⁵ kidney precursor cells derived from the wild-type mouse were transplanted into the metanephros of the mouse, from which the ureteric bud was removed. Thereafter, the organ culture of the metanephros was continued for 5 days.

### <<Tissue-Specific Removal of Metanephric Mesenchyme>>

Subsequently, tamoxifen and diphtheria toxin were added to the culture medium for organ culture. As a result, in the Six2-expressing cell, which is a metanephric mesenchyme-specific marker, Cre-ER is translocated into the nucleus and recombines to express the diphtheria toxin receptor. As a result, cells of the metanephric mesenchyme were killed by diphtheria toxin. As a result, the metanephric mesenchyme was tissue-specifically removed.

### <<Transplantation of Kidney Precursor Cells>>

Subsequently, in the same manner as in Experimental Example 2, 1×10⁵ kidney precursor cells derived from the wild-type mouse were transplanted again into the metanephros of the mouse, from which the metanephric mesenchyme was removed. Thereafter, the organ culture of the metanephros was continued for 3 to 5 days.

### <<Immunostaining>>

Subsequently, the metanephros after organ culture was fixed with 4% paraformaldehyde, and therefore a tissue section sample was prepared. Subsequently, the metanephros was immunostained with an anti-calbindin antibody for staining the ureteric bud and observed with a fluorescence microscope.

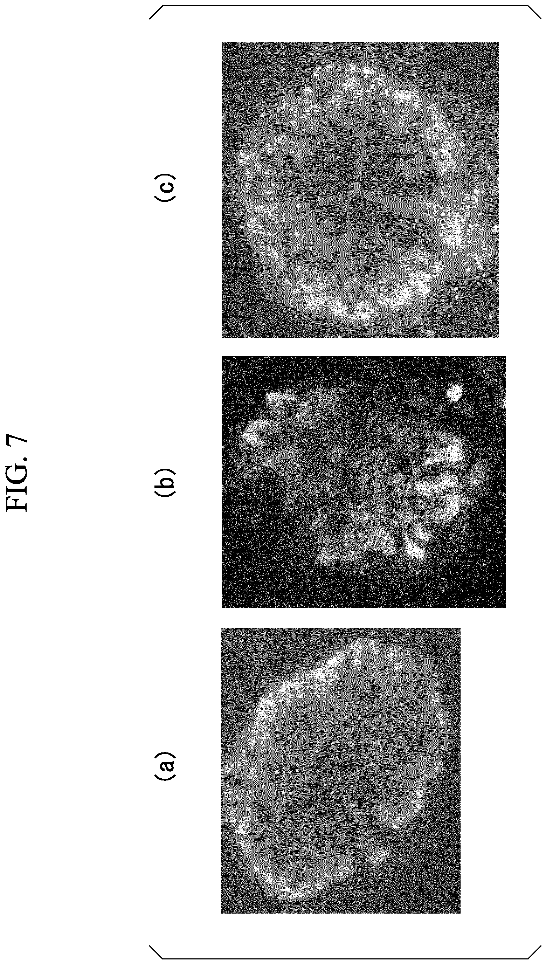

(a) of FIG. 7 is a representative photograph of the metanephros of a positive control. For the positive control, the diphtheria toxin was not added in the stage of removing the ureteric bud, and the kidney precursor cells were not transplanted. Therefore, in the positive control, tissues derived from the original ureteric bud were maintained.

(b) of FIG. 7 is a representative photograph of the metanephros of a negative control. For the negative control, the diphtheria toxin was added in the stage of removing the ureteric bud, and the kidney precursor cells were not transplanted. Accordingly, because in the negative control, the ureteric bud was tissue-specifically removed, and the kidney precursor cells were not transplanted, the ureteric bud was lost.

(c) of FIG. 7 is a representative photograph of the metanephros of a test group. In the test group, the ureteric bud was tissue-specifically removed, and the kidney precursor cells were transplanted. As a result, the ureteric bud was replaced by the transplanted kidney precursor cells, and the growth of tissues derived from the ureteric bud was observed.

Table 1 shows the results of measuring the number of ureteric bud tips in the metanephros of the positive control (n = 5), the negative control (n = 5), and the test group (n = 5).

**[Table 1]**

| | Positive control | Negative control | Test group |
|---|---|---|---|
| Number of ureteric bud tips (average value ± standard deviation) | 145.2 ± 22.2 | 73.2 ± 29.2 | 120.8 ± 23.8 |

As a result, in the negative control, a decrease in the number of ureteric bud tips was observed compared to the positive control. In contrast, in the test group, the same number of ureteric bud tips as the positive control was measured. This result shows that both metanephric mesenchyme and ureteric bud can be replaced with the transplanted kidney precursor cells by the method of the present experimental example.

### Industrial Applicability

According to the present invention, it is possible to provide a technique for producing an organ from organ precursor cells.

## Claims

1. An organ production method comprising:
tissue-specifically removing a first portion of an organ of a non-human animal partway through development thereof;
transplanting an organ precursor cell which is allogeneic or xenogeneic to the non-human animal into the organ; and
advancing development of the organ, wherein the transplanted organ precursor cell is differentiated and matured to form a part of the organ.

2. The organ production method according to claim 1, further comprising:
tissue-specifically removing a portion different from the first portion of the organ partway through the development thereof; and
transplanting an organ precursor cell which is allogeneic or xenogeneic to the non-human animal into the organ.

3. The organ production method according to claim 1 or 2,
wherein the organ precursor cell is a human cell.

4. The organ production method according to claim 1 or 2,
wherein the kidney precursor cell is a non-human animal cell.

5. The organ production method according to claim 4,
wherein the non-human animal cell is a cat cell.

6. The organ production method according to any one of claims 1 to 5,
wherein the non-human animal is a pig.

7. The organ production method according to any one of claims 1 to 5,
wherein the non-human animal is a mouse.

8. The organ production method according to any one of claims 1 to 5,
wherein the non-human animal is a cat.

9. An organ produced by the production method according to any one of claims 1 to 8.

10. An organ comprising:
a cell derived from a non-human animal; and
a cell which is allogeneic or xenogeneic to the non-human animal,
wherein a proportion of the cell which is allogeneic or xenogeneic to the non-human animal is 70% by mass or more.

11. The organ according to claim 10,
wherein the cell which is allogeneic or xenogeneic to the non-human animal is a human cell.

12. The organ according to claim 10,
wherein the cell which is allogeneic or xenogeneic to the non-human animal is a non-human animal cell.

13. The organ according to claim 12,
wherein the non-human animal cell is a cat cell.

14. A non-human animal comprising the organ according to any one of claims 9 to 13.

15. A genetically modified non-human animal for producing the organ according to any one of claims 9 to 13, from which a first portion of the organ partway through development thereof can be tissue-specifically removed.

16. The genetically modified non-human animal according to claim 15, from which a portion different from the first portion of the organ can be further tissue-specifically removed.

17. The genetically modified non-human animal according to claim 15 or 16, which is a genetically modified pig.

18. A kit for organ production comprising:
an organ of a non-human animal partway through development thereof;
a medicine which tissue-specifically removes a first portion of the organ; and
an organ precursor cell which is allogeneic or xenogeneic to the non-human animal.

19. The kit for organ production according to claim 18, further comprising:
a medicine which tissue-specifically removes a portion different from the first portion of the organ partway through the development thereof.

20. A medical drug for organ regeneration, comprising:
an organ of a non-human animal partway through development thereof;
a medicine which tissue-specifically removes a first portion of the organ; and
an organ precursor cell.

21. The medical drug for organ regeneration according to claim 20, further comprising:
a medicine which tissue-specifically removes a portion different from the first portion of the organ.
